Europäisches Patentamt

European Patent Office

Office européen des brevets

(19)

(11) Publication number: **0 035 075**
**B1**

(12) **EUROPEAN PATENT SPECIFICATION**

(45) Date of publication of patent specification: **08.02.84**

(51) Int. Cl.³: **C 07 C 43/13, C 07 C 41/06**

(21) Application number: **80300623.8**

(22) Date of filing: **29.02.80**

(54) **Process for producing ethylene glycol mono-t-butyl ether.**

(43) Date of publication of application:
**09.09.81 Bulletin 81/36**

(45) Publication of the grant of the patent:
**08.02.84 Bulletin 84/6**

(84) Designated Contracting States:
**AT BE CH DE FR GB IT NL SE**

(56) References cited:
**DE - A - 2 450 667**

(73) Proprietor: **MARUZEN PETROCHEMICAL CO., LTD.**
**25-10 Hacchobori 2-chome**
**Chuo-ku, Tokyo 104 (JP)**

(72) Inventor: **Matsumoto, Tadashi**
**No. 732, Hanaguri-cho**
**Soka-shi Saitama (JP)**
Inventor: **Kuratani, Osamu**
**No. 6132, Goi**
**Ichihara-shi Chiba (JP)**
Inventor: **Hirose, Yasunori**
**No. 732 Hanaguri-cho**
**Soka-shi Saitama (JP)**
Inventor: **Toba, Susumu**
**No. 8-30 Sattemachi Naka 3-chome**
**Kitakatsushika-gun Saitama (JP)**
Inventor: **Kamiyama, Hiroki**
**No. 902 Hanaguri-cho**
**Soka-shi Saitama (JP)**

(74) Representative: **Pearce, Anthony Richmond et al,**
**Marks & Clerk Alpha Tower Suffolk Street**
**Queensway**
**Birmingham B1 1TT (GB)**

Courier Press, Leamington Spa, England.

# O 035 075

Process for producing ethylene glycol mono-t-butyl ether

The present invention relates to a process for producing ethylene glycol mono-t-butyl ether and particularly, to a process for converting ethylene glycol di-t-butyl ether into ethylene glycol mono-t-butyl ether.

Ethylene glycol mono-t-butyl ether is a useful substance having excellent properties as a solvent, a dispersing agent and a diluent in the field of coatings, inks, etc. It is well known that ethylene glycol mono-t-butyl ether can be synthesized from isobutylene and ethylene glycol ether can be synthesized from isobutylene and ethylene glycol in the presence of an acid catalyst. It has been further described in U.S. Patents 3,317,483, 3,170,000 and 2,480,940 that, in this synthesis strongly acidic cation-exchange materials are useful as the catalyst. However, in producing ethylene glycol mono-t-butyl ether (abbreviated MBE) from isobutylene and ethylene glycol, it is not possible to avoid producing ethylene glycol di-t-butyl ether (abbreviated DBE) as a by-product, even if a catalyst is used. If ethylene glycol di-t-butyl ether is obtained as a by-product, the yield of ethylene glycol mono-t-butyl ether is reduced and this detracts from the economic value of its production. Accordingly, if it were possible to convert the ethylene glycol di-t-butyl ether by-product into the desired ethylene glycol mono-t-butyl ether, considerable advantages would be obtained. However, a satisfactory process for converting ethylene glycol di-t-butyl ether into ethylene glycol mono-t-butyl ether is not presently available.

Further, no processes have so far been discovered which effectively suppress formation of the by-product of DBE in the etherification between isobutylene and ethylene glycol.

As indicated above, U.S. Patent Nos. 3,317,483, 3,170,000 and 2,480,940 teach that ethylene glycol mono-tert-butyl ether can be synthesized from ethylene glycol and isobutylene. Of these, U.S. Patent 31700000 relates to a process of producing MBE from an olefin mixture containing isobutylene and alcohol, and selectively separating and recovering tert-olefin by the decomposition of the resulting MBE. This patent describes recycling a mixture of alcohol and MBE obtained in the final step to an etherification reactor in the first step. Also, Japanese Patent Application (OPI) No. 29413/76 describes a process of producing ethylene glycol mono-tert-butyl ether from ethylene glycol and isobutylene by previously adding ethylene glycol mono-tert-butyl ether to the reaction system. However, these processes suffer from the problem of increasing the amount of by-product ethylene glycol di-tert-butyl ether.

In the conventional processes for producing ethylene glycol mono-t-butyl ether (MBE) by using isobutylene and ethylene glycol as starting materials, the amount of ethylene glycol di-t-butyl ether (DBE) formed as a by-product is increased with the passage of reaction time. The present invention is based on the finding that MBE can be produced from DBE and ethylene glycol. The process of the present invention cannot be anticipated from the teachings of the prior art and is different from the prior art processes. Thus, the process of the invention is characterised in that DBE is present as a starting material in the reaction system when the reaction is initiated, that is, the present invention is achieved by adding DBE other than DBE formed automatically as a by-product of the reaction between ethylene glycol and any isobutylene present in the reaction system.

Accordingly, the invention resides in one aspect in a process for producing ethylene glycol mono-t-butyl ether which comprises contacting a starting mixture or feed materials comprising ethylene glycol di-t-butyl ether and ethylene glycol as starting materials with a strongly acidic cation exchange material to react ethylene glycol di-t-butyl ether in said mixture with ethylene glycol in said mixture in the presence of the strongly acidic cation-exchange material as a catalyst and produce ethylene glycol mono-t-butyl ether.

According to a further aspect, the present invention resides in a process for producing ethylene glycol mono-t-butyl ether which comprises contacting a starting mixture comprising ethylene glycol di-t-butyl ether, ethylene glycol and at least one member selected from the group consisting of ethylene glycol mono-t-butyl ether, diisobutylene and triisobutylene with a strongly acidic cation exchange material as a catalyst to react the ethylene glycol di-t-butyl ether in said mixture with the ethylene glycol in said mixture and produce ethylene glycol mono-t-butyl ether.

According to yet a further aspect, the present invention resides in a process for producing ethylene glycol mono-t-butyl ether wherein isobutylene is reacted with ethylene glycol in the presence of a strongly acidic cation-exchange material to give ethylene glycol di-t-butyl ether as a by-product, and wherein a starting mixture comprising the ethylene glycol di-t-butyl ether by-product of said reaction and ethylene glycol as starting materials is contacted with a strongly acidic cation-exchange material to react ethylene glycol di-t-butyl ether in said mixture with ethylene glycol in said mixture in the presence of the strongly acid cation exchange material as a catalyst and produce ethylene glycol mono-t-butyl ether.

According to still yet a further aspect, the present invention resides in a process for producing ethylene glycol mono-tert-butyl ether wherein ethylene glycol is reacted with isobutylene in the presence of a strongly acidic cation-exchange material, and wherein an additive comprising ethylene glycol di-tert-butyl ether is previously added to the reaction system and the reaction is conducted at a temperature of about 60 to 130°C.

The process of the present invention is particularly suitable for converting ethylene glycol di-t-

2

butyl ether formed as a by-product in the production of ethylene glycol mono-t-butyl ether from ethylene glycol and isobutylene, into ethylene glycol di-t-butyl ether. However, the ethylene glycol di-t-butyl ether used in the present invention is not necessarily limited to that produced by the reaction of isobutylene and ethylene glycol, so that ethylene glycol di-t-butyl ether produced from other raw materials and by other reactions may be used.

Further the process of the present invention is not substantially interfered with if the product, by products and additives of the reaction for producing ethylene glycol mono-t-butyl ether from isobutylene and ethylene glycol such as diisobutylene, triisobutylene, and ethylene glycol mono-t-butyl ether are present in the reaction mixture. The process for producing ethylene glycol mono-t-butyl ether from ethylene glycol and isobutylene is itself disclosed in, for example, U.S. Patent Nos: 3,170,000; 3,317,483 and 2,480,940 which comprises reacting ethylene glycol with isobutylene in the presence of a strongly acidic cation-exchange material as a catalyst. Generally, the preferred molar ratio of the feed ethylene glycol to the feed isobutylene is about 1:0.05 to 1:20 in these processes. The preferred reaction temperature is about 20 to 130°C and particularly 40 to 130°C. Although the reaction may be conducted at either atmospheric pressure or in the presence of an applied pressure, a pressure of about 1 to 50 kg/cm$_2$ is preferred. The reaction may also be carried out as either a batch or a continuous process. Typically, the reaction mixture comprises isobutylene, diisobutylene, triisobutylene, ethylene glycol, ethylene glycol mono-t-butyl ether, ethylene glycol di-t-butyl ether and the catalyst.

As the starting material of the present invention, ethylene glcyol di-t-butyl ether which contains ethylene glycol mono-t-butyl ether may be used, because it is generally easier to separate ethylene glycol di-t-butyl ether and ethylene glycol mono-t-butyl ether as a mixture from the above described reaction mixture by distillation than to separate only the by-produced ethylene glycol di-t-butyl ether, since the ethylene glycol mono-t-butyl ether and the ethylene glycol di-t-butyl ether form an azeotropic mixture. Further, since the product obtained by removing isobutylene from the above described reaction mixture and then removing part of all of the ethylene glycol mono-t-butyl ether is composed mainly of ethylene glycol di-t-butyl ether, it is possible to carry out the reaction according to the present invention by adding ethylene glycol or the catalyst (if the catalyst present or ethylene glycol present is insufficient) to convert the ethylene glycol di-t-butyl ether into ethylene glycol mono-t-butyl ether by reaction with the ethylene glycol.

As described above, the feed materials contacted with the catalyst in the process of the present invention may be a mixture of ethylene glycol and ethylene glycol di-t-butyl ether or a mixture comprising at least one member selected from ethylene glycol mono-t-butyl ether, diisobutylene and triisobutylene besides ethylene glycol di-t-butyl ether and ethylene glycol, and particularly, a mixture of ethylene glycol, ethylene glycol di-t-butyl ether and ethylene glycol mono-t-butyl ether. Amounts of diisobutylene and triisobutylene should be at most about 10% by weight and generally about 3% by weight or less in the feed materials.

In the reaction according to the present invention, the composition of the reaction product varies in accordance with the reaction temperature. Particularly, at temperatures higher than 50°C, it is preferred to limit the amount of MBE in the feed materials, because if the ethylene glycol mono-t-butyl ether is present in large amounts conversion of the di-ether into the mono-ether is inhibited.

In the present invention, the strongly acidic cation-exchange materials used as the catalyst are those which show strong acidity and are water-insoluble, namely, sulfonated materials having sulfonic acid groups (SO$_3$H) as functional groups. As the strongly acidic cation-exchange materials, there are styrene sulfonic acid type cation-exchange resins, phenol sulfonic acid type cation-exchange resins, sulfonated asphalt and sulfonated coals. The styrene sulfonic acid type cation-exchange resins are those which are prepared by sulfonating resins composed of a copolymer of styrene and an unsaturated polyene compound such as divinylbenzene (which are available as Amberlyst 15, Amberlite IR—118 and Amberlite IR—20 manufactured by Rhom & Haas Co. and Dowex 50W—X12 manufactured by Dow Chemical Co., etc.). The phenol sulfonic acid type cation-exchange resins are those which are prepared by condensing phenol-sulfonic acid with formaldehyde (which are available as Amberlite IR—1, Amberlite IR—100 and Amberlite IR—105G manufactured by Rohm & Haas Co., etc.). The sulfonated coals are those produced by sulfonating bituminous coal with sulfuric acid (which are available as Nalcite X and Nalcite AX manufactured by Dow Chemical Co. and Zeo-Karb H manufactured by the Permutit Co., Ltd., and Dursarit S manufactured by Acivit N.V., etc.). Where these strongly acidic cation-exchange materials are available as neutral materials such as the sodium salts, they should be activated by processing with a strong inorganic acid such as hydrochloric acid and washing with water prior to use to remove sodium and chloride ions. Further, as the physical structure of these cation-exchange materials, those of gel type and macroporous type materials may be used. A preferred total exchange capacity of these strongly acidic cation-exchange materials is at least about 0.5 meq/g-dry material and particularly about 1.0—7.0 meg/g.-dry material.

If the reaction temperature is too low, the reaction rate is slow. On the other hand, if it is too high, the ion-exchange catalyst can suffer thermal damage. Accordingly the preferred temperature range is about 30 to 130°C and particularly about 50 to 100°C. The reaction pressure is not particularly restricted, and the reaction can be carried out under atmospheric pressure or an applied pressure. Generally, a pressure sufficient to keep the reaction mixture at a liquid state is preferred, but a pressure

3

under which a part of the reaction mixture exists as a gas may be used. A conveniently adopted pressure is about to 150 kg/cm$^2$.

Although the molar ratio of ethylene glycol to ethylene glycol di-t-butyl ether is not particularly restricted, an excess of ethylene glycol is generally used. However, when it is not desired to have ethylene glycol in the product during separation and purification of the product, excess di-ether is used. Accordingly, the molar ratio of the feed ethylene glycol to the feed ethylene glycol di-t-butyl ether is generally in the range of about 1:0.01 to 1:20 and preferably about 1:0.05 to 1:10.

When a mixture comprising ethylene glcyol mono-t-butyl ether is used as the feed material, the molar ratio of ethylene glycol di-t-butyl ether to ethylene glycol mono-t-butyl ether in the feed material is generally in the range of about 1:0 to 1:20, preferably about 1:0 to 1:10 and particularly about 1:0 to 1:5. When the reaction temperature is high as described above, for example, 50°C or more, it is particularly preferred that the molar ratio of the di-ether to the mono-ether in the raw material is in the range of about 1:0 to 1:3, because the conversion of the di-ether into the mono-ether is obstructed, if the mono-ether is present in a large amount in the feed material. Although the amount of the cation-exchange material used as the catalyst is not necessarily limited, it is generally preferred in the range of about 0.5 to 30% and particularly 1 to 15% by weight based on the starting material. Further, although a solvent is not generally necessary, inert solvents, such as hydrocarbons exemplified by n-heptane, n-hexane, benzene, toluene, xylene, or halogenated hydrocarbons exemplified by trichloroethylene, tetrachloroethane and tetrachloromethane may be used.

The reaction can be carried out by either an agitation type batch process or a continuous process and can also be carried out in a fixed-bed flowing process. Although the reaction time is not restricted, it is preferably in the range of about 5 minutes to 50 hours and particularly about 20 minutes to 10 hours, in the case of the batch process. In the case of the continuous process, it is preferred that liquid hourly space velocity of the starting material is about 0.1 to 10 hr$^{-1}$ and particularly 0.5 to 2 hr$^{-1}$. In case of the flowing process, it is preferred that linear velocity of all starting materials passing through the catalyst bed (flow amount of the materials cm$^3$/hr based on 1 cm$^2$ of the section area of the reactor, which is represented as a liquid state at room temperature under an increased pressure) is about 30 cm/hr or more and particularly about 60 cm/hr to 50 m/hr.

The separation of ethylene glycol mono-t-butyl ether from the reaction product can be carried out by a conventional method such as distillation and/or solvent extraction using a solvent such as water, heptane or alcohol, or an extractive distillation method.

As described above, since the ethylene glycol di-tert-butyl ether can be converted into ethylene glycol mono-tert-butyl ether by reacting with ethylene glycol in the presence of the strongly acidic cation-exchange resin as a catalyst, this reaction is applicable to the production of ethylene glycol mono-tert-butyl ether by reacting isobutylene with ethylene glycol in the presence of the strongly acidic cation-exchange resin as a catalyst. That is, in the latter reaction, the ethylene glycol di-tert-butyl ether in the reaction system is converted into ethylene glycol mono-tert-butyl ether by conducting the reaction at 60 to 130°C by previously adding additives such as ethylene glcyol di-tert-butyl ether to the reaction system and, as a result, the net amount of the ethylene glycol di-tert-butyl ether formed as a by-product can be reduced.

As a result of detailed investigations of the reaction between ethylene glycol and isobutylene, it has been discovered that the reaction is unexpectedly sensitive to temperature, and that materials copresent in the reaction system influence the reaction in a different manner in a low reaction temperature (not higher than 60°C) than in a high reaction temperature (not lower than 60°C).

Thus, it has been found that, when the reaction is conducted at various temperatures within the range of 45 to 130°C by pre-adding DBE to the reaction system so as to suppress formation of the DBE by-product, the addition of DBE completely suppressed formation of the by-product of DBE over the entire temperature range. Although the yield of the end product MBE was less in the lower temperature range when adding DBE as compared with the case of not adding the additive, as the temperature increased, addition of DBE served instead to increase the end product MBE as compared with the case of not adding the DBE. In this reaction, however, the net yield of DBE in some cases reaches minus levels, though it depends upon the amount of DBE added, and hence it is sometimes necessary to separately prepare the DBE added.

When the same tests as described above were conducted by adding a mixture of DBE and MBE so as to remove the above described defect, formation of the by-product DBE was suppressed to almost zero over the entire temperature range, the yield of MBE improved with an increase in reaction temperature.

As stated above, the addition of DBE alone and the addition of the mixture of DBE and MBE tend to increase the MBE yield, with the latter providing the higher yield of MBE of the two. Thus, it might be expected that addition of MBE would further increase the yield of MBE and suppress the amount of by-produced DBE. With this in mind, the effects of the addition of MBE alone were also tested in the same manner as described above. In this test, however, it was found that the amount of by-produced DBE increased as the reaction temperature increased, the amount of the by-product seriously increased at reaction temperatures of about 60°C or above, the yield of MBE reached a maximum unexpectedly at about 70°C, and at higher reaction temperatures the yield of MBE sharply decreased.

4

Thus, in addition of DBE to the reaction system provides results substantially different from the results provided by the addition of MBE when reaction temperatures are varied and, in the case of adding a mixture of DBE and MBE, the DBE in the mixture is a dominant factor in both the formation of MBE and suppressing the formation of by-product DBE.

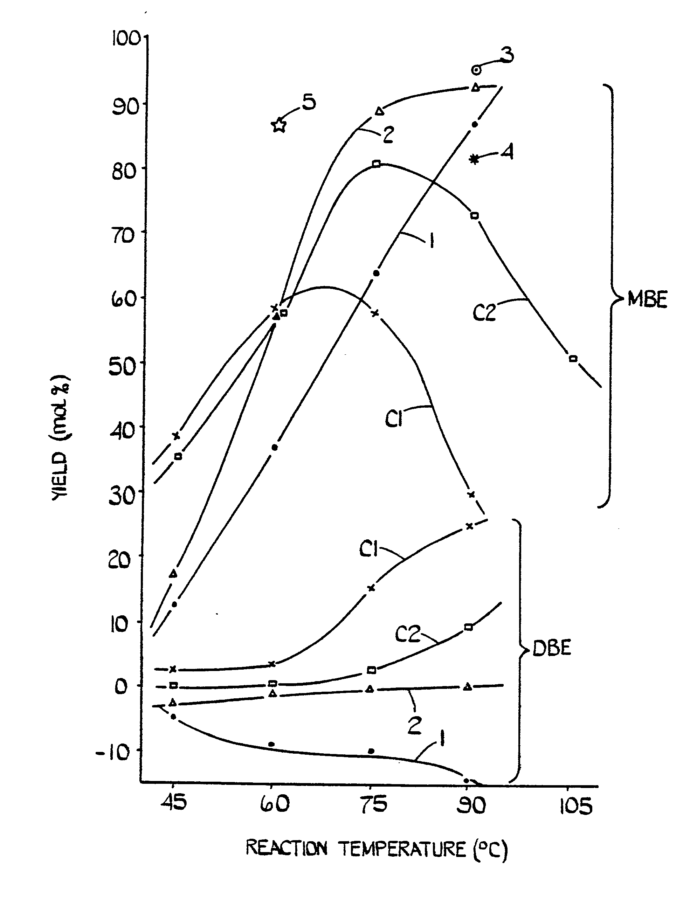

The relationship between reaction temperature and yield of MBE can be explained in more detail by referring to the accompanying drawing which is a graph showing reaction temperature plotted against the yield of ethylene glycol mono-tert-butyl ether (MBE) (mol% based on isobutylene) and the amount of the by-product of ethylene glycol di-tert-butyl ether (DBE) (mol%, based on isobutylene) in the reaction between ethylene glycol and isobutylene in the presence of a strongly acidic cation-exchange material catalyst.

In the drawing, curves 1 and 2 and points 3—5 respectively show the results in Examples 8—12 described below, and curves C1 and C2 respectively show the results in Comparative Examples 1 and 2 described below. Curve 1 and points 3 and 5 show the results of adding ethylene glycol di-tert-butyl ether to the starting materials of the reaction system, and curve 2 and point 4 show the results of using a mixture of ethylene glycol di-tert-butyl ether and ethylene glycol mono-tert-butyl ether as the additive. Curves 1, 2 and point 4 show the result of performing the reaction for 1 hour, point 3 for 3 hours, and point 5 for 4 hours. Curve C1 shows the results of using only ethylene glycol mono-tert-butyl ether as the additive, whereas curve C2 shows the results of using no additives, with the reaction time being 1 hour in both cases.

Comparison of the results of curves 1, 2, C1 and C2, which were conducted under the same reaction conditions except for additives reveals that formation of the by-product of ethylene glycol di-tert-butyl ether was effectively suppressed and the yield of the end product of ethylene glycol mono-tert-butyl ether was maintained at a high level in Examples 8 and 9 accordance with the present invention.

It will be seen from the drawing that the addition of MBE, (C1) provided the best results in the tests where the reaction was conducted for 1 hour at a temperature not higher than 60°C, whereas at a temperature higher than 60°C, the addition of no additives (C2) or the addition of a mixture of DBE and MBE (2) provided results which were better than the addition of MBE alone (C1). The MBE yield in the case of adding DBE alone (1) reached about 40% at about 60°C, which is high enough to make production practical.

At 75°C, the MBE yield with the addition of DBE alone (1) reached as high as about 60% and exceeded the yield with the addition of MBE (C1).

Further, in the tests carried out at about 85°C or above, the addition of DBE alone (1) provided better yields than that obtained without additives (C2) and substantially the same results as those obtained with the addition of a mixture of MBE and DBE (2), with the yield of MBE reaching about 90%. Additionally, in the case of no additives (C2), the yield of MBE reached a maximum at about 75°C and, as the reaction temperature exceeded 75°C, the yield of MBE sharply decreased, whereas in the case of adding MBE alone (C1), the yield of MBE reached a maximum at about 65 to 70°C and sharply decreased at reaction temperatures higher than that.

As to the amount of the by-product DBE, it can be effectively suppressed by pre-adding to the reaction system DBE (1) or a mixture of DBE and MBE (2) according to the present invention as compared with the case of pre-adding MBE (C1) or no additives (C2) to the reaction system.

The process of the present invention of reacting ethylene glycol with isobutylene to produce ethylene glycol mono-tert-butyl ether may be carried out by recycling essentially pure by-product DBE separated from the reaction mixture containing mostly ethylene glycol mono-tert-butyl ether and a slight amount of the by-product ethylene glycol di-tert-butyl ether through distillation, extraction, or like means. However, as has been stated before, ethylene glycol di-tert-butyl ether added in the process of the present invention need not be pure and, when a mixture containing MBE is used, the reaction proceeds with the DBE functioning as the dominant factor, thus much better results are obtained. In addition, separation of ethylene glycol di-tert-butyl ether and ethylene glycol mono-tert-butyl ether as a mixture from the reaction product is generally easier than separation of the by-product of ethylene glycol di-tert-butyl ether alone (because the monoether and the diether form an azeotrope), and hence addition of ethylene glycol di-tert-butyl ether containing ethylene glycol mono-tert-butyl ether to the reaction system is industrially preferable.

Isobutylene used in the process of the present invention may be isobutylene itself or a $C_4$ fraction comprising a mixture of hydrocarbons containing four carbon atoms (e.g., a mixture of isobutylene, n-butylene, butanes, etc) obtained by naphtha thermal cracking, catalytic cracking of kerosene and light oil, or the like. In the case of using such mixture, isobutylene reacts selectively.

Low reaction temperatures result in a low yield of the MBE end product, whereas too high reaction temperatures (higher than 130°C) thermally damage the catalytic activity of the ion-exchange material. Therefore, in the practice of the present invention, it is suitable to set the lower limit of the reaction temperature at about 60°C, preferablybabout 75°C, and the upper limit at about 130°C in view of heat resistance of the catalyst of ion-exchange material, with about 85 to 110°C being particularly preferable.

Reaction pressure is preferably high enough to maintain the reaction mixture in a liquid phase, but a reaction pressure permitting part of the reaction mixture to exist in a gas phase may also be employed. The reaction can be conducted under ordinary (atmospheric) pressure or an applied pressure. Usually the pressure is about 1 to 50 kg/cm².

The molar ratio of the starting ethylene glycol to the starting isobutylene is not particularly limited but is usually about 1:0.1 to 1:10, particularly about 1:0.3 to 1:5. The amount of ethylene glycol di-tert-butyl ether to be added is at least about 10 mol%, particularly preferably about 30 to 80 mol%, based on the ethylene glycol or isobutylene, whichever is less.

As has been described before, in the process of producing ethylene glycol mono-tert-butyl ether from isobutylene and ethylene glycol, the reaction conducted by adding only MBE to the reaction system results in an increased amount of the by-product of DBE and, when the reaction temperature becomes 60°C or higher, there results a decreased yield of MBE end product as compared with the case of no additives, and hence existance of a large content of MBE in the DBE added to the reaction system is not preferable. Therefore, the ratio of MBE to the DBE in the additive mixture is particularly preferably within the range of not more than about 3 mols of MBE per 1 mol of DEB, though a ratio of about 5 mols of MBE per 1 mol of DBE is still somewhat effective.

According to the process of the present invention, it is possible to convert ethylene glycol di-t-butyl ether into ethylene glycol di-t-butyl ether. Though the reaction mechanism of this reaction has not been established, ethylene glycol mono-t-butyl ether is obtained in a good yield under mild conditions. Further, since the strongly acidic cation-exchange materials used as the catalyst have a low corrosive property as compared with sulfuric acid, it is unnecessary to line the reactor or conduits with expensive anti-corrosion materials. Further, since the catalytic ion-exchange material is solid, separation of it from the reaction product is easily carried out and the reaction operation is simple.

As previously stated the conversion of ethylene glycol di-tert-butyl ether into ethylene glycol mono-tert-butyl ether by reacting the ethylene glycol di-tert-butyl ether with ethylene glycol is particularly applicable to the reaction of producing ethylene glycol mono-tert-butyl ether by reacting isobutylene with ethylene glycol. That is, when the reaction between ethylene glycol and isobutylene is conducted at high temperatures by adding ethylene glycol di-tert-butyl ether to the reaction system, formation of the by-product of ethylene glycol di-tert-butyl ether can effectively be suppressed and, at the same time, the end product of ethylene glycol mono-tert-butyl ether can be obtained in a bigh yield. In particular, unlike the reaching of U.S. Patent 3,170,000 in which MBE-containing alcohol is fed to an etherifying reactor which results in an increased amount of the by-product of DBE and a decreased yield of the end product of MBE, the process of the present invention effectively suppresses formation of the DBE by-product even when high reaction temperatures are employed and, therefore, the reaction can be completed in a short time with a high yield of ethylene glycol mono-tert-butyl ether.

According to the present invention, the net increase in the amount of ethylene glycol di-tert-butyl ether can be made zero by recycling the by-produced ethylene glycol di-tert-butyl ether to the reactor, whereby all the starting materials can be converted to ethylene glycol mono-tert-butyl ether.

The present invention will now be described in more detail by the following Examples and Comparative Examples which, however, are merely illustrative and should not be construed as limiting the process of the present invention.


Example 1

In a stainless steel autoclave having a volume of 100 ml equipped with a stirrer, 5.2 g of a styrene sulfonic acid type strongly acidic cation-exchange resin (Amberlyst 15, manufactured by Rohm & Haas Co., macroporous type resin, total exchange capacity: 4.9 meq./g-dry resin, specific surface area: 40—50 m²/g, average pore size: 200—600 Å), 0.565 mol of ethylene glycol and 0.057 mol of ethylene glycol di-t-butyl ether were mixed and a nitrogen gas was introduced thereto. The mixture was subjected to reaction at 75°C for 2 hours. The pressure was 20 kg/cm². When the liquid reaction product was analyzed, it was ascertained that the product comprised 0.520 mol of ethylene glycol, 0.004 mol of diisobutylene, 0.004 mol of ethylene glycol di-t-butyl ether and 0.098 mol of ethylene glycol mono-t-butyl ether. The result is shown in Table 1. Ethylene glycol di-t-butyl ether was converted into ethylene glycol mono-t-butyl in a good yield.


Example 2

In an autoclave having a volume of 100 ml equipped with a stirrer, 5.5 g of a styrene sulfonic acid type strongly acidic cation-exchange resin (Amberlyst 15), 0.590 mol of ethylene glycol, 0.055 mol of ethylene glycol di-t-butyl ether and 0.064 mol of ethylene glycol mono-t-butyl ether were mixed, and nitrogen gas was introduced thereto. The mixture was subjected to reaction ast 90°C for 2 hours. The pressure was 20 kg/cm². The liquid reaction product was analyzed and it was ascertained that the product comprised 0.565 mol of ethylene glycol, 0.013 mol of diisobutylene, 0.008 mol of ethylene glycol di-t-butyl ether and 0.136 mol of ethylene glycol mono-t-butyl ether. The result is shown in Table 1. In this reaction the ethylene glycol di-t-butyl ether was converted into ethylene glycol mono-t-butyl ether notwithstanding ethylene glycol mono-t-butyl ether was contained in the feed material.

## 0 035 075

Examples 3 and 4

A reaction of ethylene glycol with ethylene glycol di-t-butyl ether was carried out according to Example 1. The catalyst used was the styrene sulfonic acid type strongly acidic cation-exchange resin: Amberlite IR—118 (manufactured by Rohm & Haas Co., gel type resin, total exchange capacity: 4.4 meq/g-dry resin, specific surface area: 1 m²/g or less) in Example 3 and the styrene sulfonic acid type strongly acidic cation-exchange resin: Dowex 50 W—X12 (manufactured by Dow Chemical Co., gel type resin, total exchange capacity: 5.0 meq/g-dry resin, specific surface area: 1 m²/g or less) in Example 4. Reaction conditions and results are shown in Table 1.

TABLE 1

| Reaction temperature and time | Example 1 75°C, 2 hr | | | Example 2 90°C, 2 hr | | | Example 3 60°C, 4 hr | | | Example 4 50°C, 4 hr | | |
|---|---|---|---|---|---|---|---|---|---|---|---|---|
| Component | Feed Material (mol) | Product (mol) | (Product)-(Feed material) (mol) | Feed Material (mol) | Product (mol) | (Product)-(Feed material) (mol) | Feed Material (mol) | Product (mol) | (Product)-(Feed material) (mol) | Feed Material (mol) | Product (mol) | (Product)-(Feed material) (mol) |
| Ethylene glycol | 0.565 | 0.520 | −0.045 | 0.590 | 0.565 | −0.025 | 0.565 | 0.524 | −0.041 | 0.565 | 0.500 | −0.065 |
| Diisobutylene | 0 | 0.004 | 0.004 | 0 | 0.013 | 0.013 | 0 | 0.006 | 0.006 | 0 | 0.010 | 0.010 |
| DBE | 0.057 | 0.004 | −0.053 | 0.055 | 0.008 | −0.047 | 0.057 | 0.004 | −0.053 | 0.104 | 0.019 | −0.085 |
| MBE | 0 | 0.098 | 0.098 | 0.064 | 0.136 | 0.072 | 0 | 0.094 | 0.094 | 0 | 0.150 | 0.150 |

(Note)  DBE: Ethylene glycol di-t-butyl ether

## Example 5

A strongly acidic cation-exchange resin (Amberlyst 15) was previously swollen in ethylene glycol, and 100 ml of the swollen resin was put in a stainless steel reaction tube having a 10 mm inside diameter. After the reaction tube was heated to 75°C, 1.500 mol-hr of ethylene glycol and 0.160 mol/hr of ethylene glycol di-t-butyl ether were introduced by means of a micro-pump to carry out the reaction at 75°C. The linear velocity of the entire feed materials was about 150 cm/hr. Further, the pressure of the reaction system was maintained at 20 kg/m². When a liquid reaction product which was produced after 5 to 6 hours from beginning the reaction, at which the state of the reaction was normal, was analyzed, the results shown in the following table were obtained. The ethylene glycol di-t-butyl ether was effectively converted into ethylene glycol mono-t-butyl ether.

### TABLE 2

| Component | Feed Material | Product | (Product) -(Feed material) |
|---|---|---|---|
| | (mol/hr) | (mol/hr) | (mol/hr) |
| Ethylene glycol | 1.500 | 1.381 | −0.119 |
| Diisobutylene | 0 | 0.011 | 0.011 |
| DBE | 0.160 | 0.019 | −0.141 |
| MBE | 0 | 0.260 | 0.260 |

## Example 6

The reaction was carried out in the same manner as in Example 1 except that a mixture comprising 0.220 mol of ethylene glycol mono-t-butyl ether and small amounts of diisobutylene, triisobutylene and water besides 0.590 mol of ethylene glycol and 0.055 mol of ethylene glycol di-t-butyl ether was used as the feed material. The catalyst used was the strongly acidic cation-exchange resin (Amberlyst 15). The reaction conditions and the result of analyzing the liquid reaction product are shown in the following table.

### TABLE 3

| Reaction temperature and time | 75°C, 2 hr | | |
|---|---|---|---|

| Component | Feed Material | Product | (Product) -(Feed Material |
|---|---|---|---|
| | (mol) | (mol) | (mol) |
| Ethylene glycol | 0.590 | 0.562 | −0.028 |
| Diisobutylene | 0.002 | 0.011 | 0.009 |
| Triisobutylene | 0.001 | 0.001 | 0 |
| DBE | 0.055 | 0.002 | −0.053 |
| MBE | 0.220 | 0.301 | 0.081 |

## Example 7

In an autoclave having a 100 ml volume equipped with a stirrer, 5.5 g of sulfonated coal: Dusarit S (manufactured by Activit N.V. Co.), 0.565 mol of ethylene glycol and 0.057 mol of ethylene lycol di-t-butyl ether were mixed, and nitrogen gas was introduced thereto. The mixture was subjected to reaction at 60°C for 4 hours. The pressure was 20 kg/cm². The result of analyzing the liquid reaction product is shown in the following table. The ethylene glycol di-t-butyl ether was effectively converted into ethylene glycol mono-t-butyl ether.

TABLE 4

| Reaction temperature and time | | 60°C, 4 hr | |
|---|---|---|---|
| Component | Feed Material | Product | (Product) -(Feed material) |
| | (mol) | (mol) | (mol) |
| Ethylene glycol | 0.565 | 0.542 | −0.023 |
| Diisobutylene | 0 | 0.002 | 0.002 |
| DBE | 0.057 | 0.031 | −0.026 |
| MBE | 0 | 0.049 | 0.049 |

Example 8

5.25 g of Amberlyst 15 (made by Rohm & Haas Co.; macroporous type resin; total exchange capacity: 4.9 meq/g-dry resin; specific surface area: 40—50 m²/g; average pore size: 200—600 Å) as a catalyst and, as starting materials, 35.0 g (0.565 mol) of ethylene glycol, 11.7 g (0.209 mol) of isobutylene, and as an additive 17.9 g (0.103 mol) of ethylene glycol di-tert-butyl ether were charged in an autoclave (200 ml in volume) equipped with a stirrer rotating at 1,300 rpm, followed by charging with nitrogen gas and reacting for 1 hour under a pressure of 20 kg/cm² at a predetermined reaction temperature. After completion of the reaction, isobutylene was discharged, and the remaining reaction product was analyzed according to gas chromatograph. The results thus obtained are tabluated in Table 5.

Ethylene glycol mono-tert-butyl ether was produced in a high yield, and the formation of ethylene glycol di-tert-butyl ether was effectively suppressed.

TABLE 5

| | Reaction Temperature (°C) | | | |
| --- | --- | --- | --- | --- |
| | 45 | 60 | 75 | 90 |
| Ethylene Glycol (mol) Feed Amount (a) | 0.565 | 0.565 | 0.565 | 0.565 |
| Resulting Amount (b) | 0.549 | 0.506 | 0.452 | 0.413 |
| Yield (b—a) | —0.016 | —0.059 | —0.113 | —0.152 |
| Diisobutylene | | | | |
| Yield (mol) | 0.001 | 0.009 | 0.006 | 0.005 |
| DBE (mol) | | | | |
| Feed Amount (a) | 0.103 | 0.103 | 0.103 | 0.103 |
| Resulting Amount (b) | 0.093 | 0.083 | 0.082 | 0.073 |
| Yield (b—a) | —0.011 | —0.019 | —0.021 | —0.030 |
| Yield (b—a) (mol%*) | —5.0 | —9.1 | —9.9 | —14.4 |
| MBE (mol) | | | | |
| Feed Amount (a) | 0 | 0 | 0 | 0 |
| Resulting Amount (b) | 0.027 | 0.078 | 0.134 | 0.182 |
| Yield (b—a) | 0.027 | 0.078 | 0.134 | 0.182 |
| " (mol%*) | 12.7 | 37.1 | 64.0 | 87.0 |
| Isobutylene | | | | |
| Feed Amount (mol) | 0.209 | 0.209 | 0.209 | 0.209 |

(Note) *based on isobutylene

Example 9

The same procedures as described in Example 8 were conducted except using a mixture of 17.9 g of ethylene glycol di-tert-butyl ether and 24.8 g (0.210 mol) of ethylene glycol mono-tert-butyl ether in place of 17.9 g of ethylene glycol di-tert-butyl ether to obtain the results shown in Table 6. The MBE end product was produced in high yield, and the net amount of DBE produced was zero or slightly minus.

11

**O O35 O75**

TABLE 6

| | Reaction Temperature (°C) | | | |
|---|---|---|---|---|
| | 45 | 60 | 75 | 90 |
| Ethylene Glycol (mol) | | | | |
| Feed Amount (a) | 0.565 | 0.565 | 0.565 | 0.565 |
| Resulting Amount (b) | 0.535 | 0.447 | 0.380 | 0.371 |
| Yield (b—a) | −0.030 | −0.118 | −0.184 | −0.193 |
| Diisobutylene | | | | |
| Yield (mol) | 0.008 | 0.017 | 0.014 | 0.008 |
| DBE (mol) | | | | |
| Feed Amount (a) | 0.103 | 0.103 | 0.103 | 0.103 |
| Resulting Amount (b) | 0.097 | 0.101 | 0.102 | 0.103 |
| Yield (b—a) | −0.006 | −0.002 | −0.001 | 0.000 |
| ,, (mol%) | −2.9 | −1.1 | −0.5 | 0.000 |
| MBE (mol) | | | | |
| Feed Amount (a) | 0.210 | 0.210 | 0.210 | 0.210 |
| Resulting Amount (b) | 0.246 | 0.330 | 0.395 | 0.403 |
| Yield (b—a) | 0.036 | 0.120 | 0.185 | 0.193 |
| ,, (mol%) | 17.2 | 57.2 | 88.7 | 92.4 |
| Isobutylene | | | | |
| Feed Amount (mol) | 0.209 | 0.209 | 0.209 | 0.209 |

Comparative Example 1

The same procedures as described in Example 8 were conducted except using 24.8 g of ethylene glycol mono-tert-butyl ether in place of 17.9 g of ethylene glycol di-tert-butyl ether to obtain the results shown in Table 7. The yield of MBE end product reached a maximum at about 65—70°C. Formation of the by-product of DBE was considerable at about 60°C or above.

### TABLE 7

| | Reaction Temperature (°C) | | | |
| --- | --- | --- | --- | --- |
| | 45 | 60 | 75 | 90 |
| Ethylene Glycol (mol) | | | | |
| Feed Amount (a) | 0.565 | 0.565 | 0.565 | 0.565 |
| Resulting Amount (b) | 0.479 | 0.437 | 0.413 | 0.451 |
| Yield (b-a) | −0.086 | −0.128 | −0.152 | −0.113 |
| Diisobutylene | | | | |
| Yield (mol) | 0.004 | 0.011 | 0.010 | 0.009 |
| DBE (mol) | | | | |
| Feed Amount (a) | 0 | 0 | 0 | 0 |
| Resulting Amount (b) | 0.005 | 0.007 | 0.032 | 0.052 |
| Yield (b-a) | 0.005 | 0.007 | 0.032 | 0.052 |
| „ (mol%) | 2.5 | 3.2 | 15.1 | 24.7 |
| MBE (mol) | | | | |
| Feed Amount (a) | 0.210 | 0.210 | 0.210 | 0.210 |
| Resulting Amount (b) | 0.291 | 0.331 | 0.330 | 0.272 |
| Yield (b-a) | 0.081 | 0.121 | 0.120 | 0.062 |
| „ (mol%) | 38.6 | 58.0 | 57.7 | 29.6 |
| Isobutylene | | | | |
| Feed Amount (mol) | 0.209 | 0.209 | 0.209 | 0.209 |

Comparative Example 2

The same procedures as in Example 8 were conducted except for using only ethylene glycol and isobutylene to obtain the results shown in Table 8. The yield of the end product of MBE reached a maximum at about 75°C.

TABLE 8

| | Reaction Temperature (°C) | | | | |
|---|---|---|---|---|---|
| | 45 | 60 | 75 | 90 | 105 |
| Ethylene Glycol (mol) | | | | | |
| Feed Amount (a) | 0.565 | 0.565 | 0.565 | 0.565 | 0.565 |
| Resulting Amount (b) | 0.490 | 0.444 | 0.390 | 0.393 | |
| Yield (b—a) | —0.074 | —0.121 | —0.174 | —0.172 | |
| Diisobutylene | | | | | |
| Yield (mol) | 0.001 | 0.007 | 0.005 | 0.004 | |
| DBE (mol) | | | | | |
| Feed Amount (a) | 0 | 0 | 0 | 0 | 0 |
| Resulting Amount (b) | 0 | 0.001 | 0.006 | 0.020 | |
| Yield (b-a) | 0 | 0.001 | 0.006 | 0.020 | |
| „ (mol%) | 0.0 | 0.3 | 2.6 | 9.5 | |
| MBE (mol) | | | | | |
| Feed Amount (a) | 0 | 0 | 0 | 0 | 0 |
| Resulting Amount (b) | 0.074 | 0.120 | 0.169 | 0.152 | 0.106 |
| Yield (b—a) | 0.074 | 0.120 | 0.169 | 0.152 | 0.106 |
| „ (mol%) | 35.5 | 57.5 | 80.7 | 72.8 | 50.7 |
| Isobutylene | | | | | |
| Feed Amount (mol) | 0.209 | 0.209 | 0.209 | 0.209 | 0.209 |

Examples 10—12

The same procedures as in Example 8 were conducted using an autoclave (200 ml in content volume) equipped with a stirrer and reacting the starting materials under reaction conditions shown in Table 9. The results thus obtained are shown in Table 9.

TABLE 9

| | Example 10 | Example 11 | Example 12 |
|---|---|---|---|
| Reaction Temperature and Time | 90°C, 3 hr | 90°C, 1 hr | 60°C, 4 hr |
| **Component** | | | |
| **Ethylene Glycol (mol)** | | | |
| Feed Amount (a) | 0.555 | 0.565 | 0.565 |
| Resulting Amount (b) | 0.356 | 0.404 | 0.384 |
| Yield (b—a) | —0.199 | —0.161 | —0.181 |
| **Diisobutylene** | | | |
| Yield (mol) | 0 | 0.006 | 0.004 |
| **DBE (mol)** | | | |
| Feed Amount (a) | 0.051 | 0.067 | 0.043 |
| Resulting Amount (b) | 0.051 | 0.060 | 0.043 |
| Yield (b—a) | 0 | —0.007 | 0 |
| „ (mol%) | 0.0 | —3.4 | 0.0 |
| **MBE (mol)** | | | |
| Feed Amount (a) | 0 | 0.104 | 0 |
| Resulting Amount (b) | 0.199 | 0.272 | 0.181 |
| Yield (b—a) | 0.199 | 0.168 | 0.181 |
| „ (mol%) | 95.2 | 81.6 | 87.0 |
| **Isobutylene** | | | |
| Feed Amount (mol) | 0.209 | 0.206 | 0.208 |

From the results of Examples 10 and 12, it will be seen that the net amount of by-produced DBE can be maintained zero by adding only DBE and controlling of the DBE amount added, to thereby obtain MBE in a high yield.

In Example 11, the amounts of DBE and MBE added respectively were reduced to about $\frac{1}{2}$ as compared with Example 9. In this Example, too, the effect of preventing formation of the by-product of DBE was observed, though the yield of MBE was slightly less than in Example 9.

Yields of MBE and DBE obtained in Examples 8 and 9 and Comparative Examples 1 and 2 and yields of MBE in Examples 10 to 12 are shown in the attached drawing.

Example 13

100 ml of Amberlyst 15 previously swollen with ethylene glycol was charged into a stainless steel-made pressure-resistant pipe type reactor. A $C_4$ fraction obtained by naphtha cracking (isobutylene content: 45.0 wt%), ethylene glycol, and ethylene glycol di-tert-butyl ether were continuously fed to a catalyst bed under the reaction conditions of 75°C, 20 kg-cm². Linear velocity of the total starting materials and additive was 484 cm/hr. Reaction results of steady state which was

# O 035 075

attained five hours after initiation of the reaction are shown in Table 10. There was observed reduction in the amount of ethylene glycol di-tert-butyl ether.

## Comparative Example 3

Table 10 shows the results obtained by repeating the procedure of Example 13 except for adding no ethylene glycol di-tert-butyl ether to the starting material. Unlike Example 13, ethylene glycol di-tert-butyl ether was by-produced.

Additionally, feed amounts, resulting amounts, and yields in Table 10 are presented as the amounts fed or produced in the unit of mole per 1 hr per 1 ml of the catalyst.

## TABLE 10

|  | Example 13 | Comparative Example 3 |
|---|---|---|
| Reaction Temperature (°C) | 75 | 75 |
| **Component** | | |
| Ethylene Glycol | | |
| Feed Amount (a) | 22.50 | 22.50 |
| Resulting Amount (b) | 17.90 | 16.53 |
| Yield (b—a) | —4.60 | —5.97 |
| Diisobutylene | | |
| Yield | 0.05 | 0.05 |
| DBE | | |
| Feed Amount (a) | 1.04 | 0 |
| Resulting Amount (b) | 0.70 | 0.62 |
| Yield (b—a) | —0.34 | 0.62 |
| „ (mol%) | —4.3 | 7.9 |
| MBE | | |
| Feed Amount (a) | 0 | 0 |
| Resulting Amount (b) | 4.94 | 5.35 |
| Yield (b—a) | 4.94 | 5.35 |
| „ (mol%) | 62.7 | 67.9 |
| Isobutylene | | |
| Feed Amount | 7.88 | 7.88 |

## Example 14

The reaction was conducted in the same manner as in Example 9 except for using as a catalyst 5.3 g of styrene sulfonic acid type strongly acidic cation-exchange resin, Amberlite IR-118 (made by Rohm & Haas Co.; gel type resin; total exchange capacity: 4.4 meq/g/dry resin; specific surface area: not more than 1 $m^2/g$) previously well dried at 120°C under reduced pressure. Reaction conditions were 75°C for the reaction temperature and 1 hour for the reaction time. Results of the reaction are shown in Table 11. As shown in Table 11, the end product of ethylene glycol mono-tert-butyl ether was

16

produced in a high yield and the net amount of by-produced ethylene glycol di-tert-butyl ether was zero.

Example 15

The reaction was conducted in the same manner as in Example 9 except for using 5.5 g of commercially available sulfonated coal, Dusarit S (made by Activit N.V.) as a catalyst and reacting for 3 hours. The reaction temperature was 75°C. Results thus obtained are shown in Table 11.

As can be seen from Table 11, the yield of the end product of ethylene glycol mono-tert-butyl ether was high, and the net amount of ethylene glycol di-tert-butyl ether was slightly decreased.

TABLE 11

| | | Example 14 | Example 15 |
|---|---|---|---|
| Reaction Temperature and Time | | 75°C, 1 hr | 75°C, 3 hr |
| **Component** | | | |
| Ethylene Glycol (mol) | | | |
| | Feed Amount (a) | 0.565 | 0.565 |
| | Resulting Amount (b) | 0.381 | 0.390 |
| | Yield (b—a) | —0.184 | —0.175 |
| Diisobutylene | | | |
| | Yield (mol) | 0.012 | 0.010 |
| DBE (mol) | | | |
| | Feed Amount (a) | 0.103 | 0.103 |
| | Resulting Amount (b) | 0.103 | 0.102 |
| | Yield (b—a) | 0.000 | —0.001 |
| | „ (mol%) | 0.0 | —0.5 |
| MBE (mol) | | | |
| | Feed Amount (a) | 0.210 | 0.210 |
| | Resulting Amount (b) | 0.394 | 0.386 |
| | Yield (b—a) | 0.184 | 0.176 |
| | „ (mol%) | 88.0 | 84.2 |
| Isobutylene | | | |
| | Feed Amount (mol) | 0.209 | 0.209 |

Example 16

35.0 g of ethylene glycol, 9.9 g of DBE and 5.0 g of amberlyst 15 were charged in a stainless autoclave (100 milliliter in volume) followed by conducting the reaction at 60°C. The reaction product was analysed and the changes in the amounts (mol percent) of the components ethylene glycol, MBE and DBE with the passage of time are tabulated in Table 12. The compositions (mol percent) as described in Table 12 are based on the total moles (100 mol percent) of ethylene glycol, MBE and DBE in the reaction product in which the butane, butene and polymer of butene formed as by-product had been removed.

17

# O 035 075

The results as described in Table 12 show that the amount of each of DBE and ethylene glycol is reduced with the passage of time and the amount of MBE produced is increased with the passage of time. Therefore, it can be appreciated from the results of Table 12 that the reaction of converting DBE into MBE is caused by reaction of DBE with ethylene glycol.

TABLE 12

| Reaction Time (HR) | 0 | 1 | 2 | 4 | 5 |
|---|---|---|---|---|---|
| Component | | | | | |
| EG (mol percent) | 90.8 | 88.6 | 87.4 | 84.0 | 83.8 |
| MBE (mol percent) | 0 | 4.8 | 8.8 | 15.4 | 16.0 |
| DBE (mol percent) | 9.2 | 6.6 | 3.8 | 0.6 | 0.2 |

(note) EG: Ethylene Glycol

Comparative Example 4

11.8 g of isobutylene, 35.0 g of ethylene glycol and 5.0 g of amberlyst 15 were charged in a stainless autoclave (100 milli-liter in volume), followed by conducting the reaction at 60°C. Following analysis of the reaction product in the same way as in Example 16, the changes in the amount (mol percent) of ethylene glycol, MBE and DBE with the passage of time are tabulated in Table 13.

The results of Table 13 show that the amount of DBE is increased with the passage of time. Further, the amount of the MBE produced reached a maximum at reaction time of about 3 hours, and then gradually reduced. Therefore, it can be appreciated from the results of Table 13 that the reaction comprising producing MBE by reacting ethylene glycol with isobutylene and then producing DBE by reacting the resulting MBE with isobutylene is successively conducted.

TABLE 13

| Reaction Time (HR) | 0 | 1 | 2.5 | 4 | 6 |
|---|---|---|---|---|---|
| EG (mol percent) | 100 | 78.6 | 70.4 | 70.1 | 69.4 |
| MBE (mol percent) | 0 | 21.2 | 27.5 | 26.9 | 25.5 |
| DBE (mol percent) | 0 | 0.2 | 2.1 | 3.0 | 5.1 |

Note: Mol percent: Based on the total amount (mol) of EG, MBE and DBE.

## Claims

1. A process for producing ethylene glycol mono-t-butyl ether using ethylene glycol as one of the starting materials and a strongly acidic cation exchange material as a catalyst, characterized in that a starting mixture comprising ethylene glycol di-t-butyl ether and said ethylene glycol is contacted with said strongly acidic cation exchange material to react ethylene glycol di-t-butyl ether in said mixture with ethylene glycol in said mixture and produce ethylene glycol mono-t-butyl ether.

2. A process for producing ethylene glycol mono-t-butyl ether using ethylene glycol as one of the starting materials and a strongly acidic cation exchange material as a catalyst, characterized in that a starting mixture comprising ethylene glycol di-t-butyl ether, said ethylene glycol and at least one member selected from ethylene glycol mono-t-butyl ether, diisobutylene and triisobutylene is contacted with said acidic cation exchange material to react the ethylene glycol di-t-butyl ether in said mixture with the ethylene glycol in said mixture and produce ethylene glycol mono-t-butyl ether.

3. A process as claimed in claim 2, wherein the starting material is a mixture of ethylene glycol di-t-butyl ether, ethylene glycol and ethylene glycol mono-t-butyl ether.

4. A process as claimed in any preceding claim wherein the molar ratio of the feed ethylene glycol to the feed ethylene glycol di-t-butyl ether is 1:0.01 to 1:20.

18

5. A process as claimed in claim 2 or claim 3 wherein the molar ratio of the feed ethylene glycol di-t-butyl ether to the feed ethylene glycol mono-t-butyl ether is 1:0 to 1:20.

6. A process as claimed in claim 2 or claim 3, wherein the molar ratio of the feed ethylene glycol di-t-butyl ether to the feed ethylene glycol mono-t-butyl is 1:0 to 1:5.

7. A process as claimed in any preceding claim, wherein the reaction is carried out at 30 to 130°C.

8. A process as claimed in claim 1, comprising using, as said ethylene glycol di-t-butyl ether, ethylene glycol di-t-butyl ethyl obtained as a by-product from the reaction of isobutylene with ethylene glycol in the presence of a strongly acidic cation exchange material as a catalyst.

9. A process as claimed in claim 8, wherein said ethylene glycol di-t-butyl ether by-product is present as a mixture with at least one member selected from ethylene glycol mono-t-butyl ether, diisobutylene, and triisobutylene.

10. A process as claimed in claim 9, wherein said ethylene glycol di-t-butyl ether by-product is removed from said mixture as an azeotrope with ethylene glycol mono-t-butyl ether prior to the reaction to produce ethylene glycol mono-t-butyl ether.

11. A process for producing ethylene glycol mono-tert-butyl ether wherein ethylene glycol is reacted with isobutylene in the presence of a strongly acidic cation-exchange material, and characterized in that an additive comprising ethylene glycol di-tert-butyl ether is previously added to the reaction system and the reaction is conducted at a temperature of 60 to 130°C.

12. A process as claimed in claim 11, wherein said additive is a mixture of ethylene glycol di-tert-butyl ether and ethylene glycol mono-tert-butyl ether.

13. A process as claimed in claim 12, wherein the molar ratio of ethylene glycol di-tert-butyl ether to ethylene glycol mono-tert-butyl ether in said mixture is 1:0 to 1:5.

14. A process as claimed in claim 12, wherein the molar ratio of ethylene glycol di-tert-butyl ether to ethylene glycol mono-tert-butyl ether in said mixture is 1:0 to 1:3.

15. A process as claimed in any one of claims 11 to 14, wherein said reaction is carried out in the presence of diisobutylene or triisobutylene.

16. A process as claimed in any one of claims 11 to 15, wherein a mixture of hydrocarbons having four carbon atoms and containing isobutylene is used as the isobutylene component which is reacted with the ethylene glycol.

17. A process as claimed in any one of claims 11 to 16, wherein the molar ratio of ethylene glycol to isobutylene is 1:0.1 to 1:10.

18. A process as claimed in any one of claims 11 to 17, wherein the ethylene glycol di-tert-butyl ether is added in an amount of at least 10 mol% based on ethylene glycol or isobutylene, whichever is less.

19. A process as claimed in any one of claims 11 to 18, wherein the ethylene glycol di-tert-butyl ether is added in an amount of 30 to 80 mol% based on ethylene glycol or isobutylene, whichever is less.

20. A process as claimed in any one of claims 11 to 19 wherein said temperature is 75 to 130°C.

21. A process as claimed in any one of claims 11 to 20, wherein said temperature is 85 to 110°C.

22. A process as claimed in any preceding claim, wherein the reaction is conducted as a batch process.

23. A process as claimed in any one of claims 1 to 21, wherein the reaction is conducted as a continuous flow process.

24. A process as claimed in claim 23, wherein the linear velocity of the starting materials in the reaction is at least 30 cm/hr.

25. A process as claimed in any preceding claim wherein the cation exchange material is a sulfonated material.

26. A process as claimed in claim 25, wherein said cation-exchange material is a styrene sulfonic acid type cation-exchange resin, a phenolsulfonic acid type cation-exchange resin, a sulfonated coal or a sulfonated asphalt.

**Patentansprüche**

1. Verfahren zur Herstellung von Ethylenglykol-mono-t-butylether unter Verwendung von Ethylenglykol als eines der Ausgangsmaterialien und eines stark sauren Kationenaustauschmaterials als Katalysator, dadurch gekennzeichnet, dass eine Ausgangsmischung aus Ethylenglykol-di-t-butylether und dem Ethylenglykol mit dem stark sauren Kationenaustauschmaterial in Berührung gebracht wird, unter Umsetzung von Ethylenglykol-di-t-butylether in dem Gemisch mit Ethylenglykol in dem Gemisch und unter Bildung von Ethylenglykol-mono-t-butylether.

2. Verfahren zur Herstellung von Ethylenglykol-mono-t-butylether unter Verwendung von Ethylenglykol als eines der Ausgangsmaterialien und eines stark sauren Kationenaustauschmaterials als Katalysator, dadurch gekennzeichnet, dass man als Ausgangsmischung eine Mischung aus Ethylenglykol-di-t-butylether, dem Ethylenglykol und wenigstens einem Glied ausgewählt aus Ethylenglykol-mono-t-butylether, Diisobutylen und Triisobutylen verwendet und mit dem stark sauren Kationenaustauschmaterial in Berührung bringt, unter Umsetzung von Ethylenglykol-di-t-butylether in dem Gemisch mit dem Ethylenglykol in dem Gemisch und unter Bildung von Ethylenglykol-mono-t-butyl-ether.

3. Verfahren gemäss Anspruch 2, dadurch gekennzeichnet, dass das Ausgangsmaterial eine Mischung aus Ethylenglykol-di-t-butylether, Ethylenglykol und Ethylenglykol-mono-t-butylether ist.

4. Verfahren gemäss einem der vorhergehenden Ansprüche, dadurch gekennzeichnet, dass das Molverhältnis des zugeführten Ethylenglykols zu dem zugeführten Ethylenglykol-di-t-butylether 1:0,01 bis 1:20 beträgt.

5. Verfahren gemäss Ansprüchen 2 oder 3, dadurch gekennzeichnet, dass das Molverhältnis des zugeführten Ethylenglykol-di-t-butylethers zu dem zugeführten Ethylenglykol-mono-t-butylether 1:0 bis 1:20 beträgt.

6. Verfahren gemäss Ansprüchen 2 oder 3, dadurch gekennzeichnet, dass das Molverhältnis des zugeführten Ethylenglykol-di-t-butylethers zu dem zugeführten Ethylenglykol-mono-t-butylether 1:0 bis 1:5 beträgt.

7. Verfahren gemäss einem der vorhergehenden Ansprüche, dadurch gekennzeichnet, dass die Umsetzung bei 30 bis 130°C durchgeführt wird.

8. Verfahren gemäss Anspruch 1, dadurch gekennzeichnet, dass man als Ethylenglykol-di-t-butylether einen Ethylenglykol-di-t-butylether verwendet, der als Nebenprodukt aus der Umsetzung von Isobutylen mit Ethylenglykol in Gegenwart eines stark sauren Kationenaustauschmaterials als Katalysator erhalten wurde.

9. Verfahren gemäss Anspruch 8, dadurch gekennzeichnet, dass das Ethylenglykol-di-t-butylether-Nebenprodukt in einer Mischung mit wenigstens einem Glied, ausgewählt aus Ethylenglykol-mono-t-butylether, Diisobutylen und Triisobutylen vorliegt.

10. Verfahren gemäss Anspruch 9, dadurch gekennzeichnet, dass das Ethylenglykol-di-t-butylether-Nebenprodukt aus der Mischung als ein Azeotrop mit Ethylenglykol-mono-t-butylether vor der Umsetzung zur Bildung von Ethylenglykol-mono-t-butylether entfernt wird.

11. Verfahren zur Herstellung von Ethylenglykol-mono-t-butylether, bei dem man Ethylenglykol mit Isobutylen in Gegenwart eines stark sauren Kationenaustauschmaterials umsetzt, dadurch gekennzeichnet, dass man zuvor ein Additiv aus Ethylenglykol-di-t-butylether zu dem Reaktionssystem zugibt und die Umsetzung bei einer Temperatur von 60 bis 130°C durchführt.

12. Verfahren gemäss Anspruch 11, dadurch gekennzeichnet, dass das Additiv eine Mischung aus Ethylenglykol-di-t-butylether und Ethylenglykol-mono-t-butylether ist.

13. Verfahren gemäss Anspruch 12, dadurch gekennzeichnet, dass das Molverhältnis von Ethylenglykol-di-t-butylether zu Ethylenglykol-mono-t-butylether in dem Gemisch 1:0 bis 1:5 beträgt.

14. Verfahren gemäss Anspruch 12, dadurch gekennzeichnet, dass das Molverhältnis von Ethylenglykol-di-t-butylether zu Ethylenglykol-mono-t-butylether in dem Gemisch 1:0 bis 1:3 beträgt.

15. Verfahren gemäss einem der Ansprüche 11 bis 14, dadurch gekennzeichnet, dass die Umsetzung in Gegenwart von Diisobutylen oder Triisobutylen durchgeführt wird.

16. Verfahren gemäss einem der Ansprüche 11 bis 15, dadurch gekennzeichnet, dass eine Mischung aus Kohlenwasserstoffen mit 4 Kohlenstoffatomen, enthaltend Isobutylen, als Isobutylenkomponente, die mit dem Ethylenglykol umgesetzt wird, verwendet wird.

17. Verfahren gemäss einem der Ansprüche 11 bis 16, dadurch gekennzeichnet, dass das Molverhältnis von Ethylenglykol zu Isobutylen 1:0,1 bis 1:10 beträgt.

18. Verfahren gemäss einem der Ansprüche 11 bis 17, dadurch gekennzeichnet, dass der Ethylenglykol-di-t-butylether in einer Menge von wenigstens 10 Mol.%, bezogen auf Ethylenglykol oder Isobutylen, je nachdem welches in geringerer Menge vorliegt, zugegeben wird.

19. Verfahren gemäss einem der Ansprüche 11 bis 18, dadurch gekennzeichnet, dass der Ethylenglykol-di-t-butylether in einer Menge von 30 bis 80 Mol.%, bezogen auf Ethylenglykol oder Isobutylen, je nachdem, welches in geringerer Menge vorliegt, zugegeben wird.

20. Verfahren gemäss einem der Ansprüche 11 bis 19, dadurch gekennzeichnet, dass die Temperatur 75 bis 130°C beträgt.

21. Verfahren gemäss einem der Ansprüche 11 bis 20, dadurch gekennzeichnet, dass die Temperatur 85 bis 110°C beträgt.

22. Verfahren gemäss einem der vorhergehenden Ansprüche, dadurch gekennzeichnet, dass die Umsetzung absatzweise durchgeführt wird.

23. Verfahren gemäss einem der Ansprüche 1 bis 21, dadurch gekennzeichnet, dass die Umsetzung als ein kontinuierliches Fliessverfahren durchgeführt wird.

24. Verfahren gemäss Anspruch 23, dadurch gekennzeichnet, dass die Lineargeschwindigkeit der Ausgangsmaterialien bei der Umsetzung mindestens 30 cm/h beträgt.

25. Verfahren gemäss einem der vorhergehenden Ansprüche dadurch gekennzeichnet, dass das Kationenaustauschmaterial ein sulfoniertes Material ist.

26. Verfahren gemäss Anspruch 25, dadurch gekennzeichnet, dass das Kationenaustauschmaterial ein Kationenaustauschharz vom Styrolsulfonsäuretyp, ein Kationenaustauschharz vom Phenolsulfonsäuretyp, eine sulfonierte Kohle oder ein sulfoniertes Asphalt ist.

# 0 035 075

**Revendications**

1. Procédé de préparation de l'éther mono-tert-butylique de l'éthylène glycol utilisant l'éthylène glycol comme l'un des matériaux de départ et un échangeur de cations fortement acide comme catalyseur, caractérisé en ce que l'on met un mélange de départ comportant de l'éther di-tert-butylique d'éthylène glycol et ledit éthylène glycol au contact dudit échangeur de cations fortement acide afin de faire réagir l'éther di-tert-butylique d'éthylène glycol dans ledit mélange avec l'éthylène glycol et d'obtenir l'éther mono-tert-butylique d'éthylène glycol.

2. Procédé de préparation de l'éther mono-tert-butylique d'éthylène glycol utilisant l'éthylène glycol comme l'un des matériaux de départ et un échangeur de cations fortement acide comme catalyseur, caractérisé en ce que l'on met un mélange de départ comportant de l'éther di-tert-butylique d'éthylène glycol, ledit éthylène glycol et au moins un produit choisi parmi l'éther mono-tert-butylique d'éthylène glycol, le diisobutylène et le triisobutylène au contact dudit échangeur de cations fortement acide afin de faire réagir l'éther di-tert-butylique d'éthylène glycol dans ledit mélange avec l'éthylène glycol dans ledit mélange et d'obtenir l'éther mono-tert-butylique d'éthylène glycol.

3. Procédé selon la Revendication 2 selon lequel le matériau de départ est un mélange d'éther di-tert-butylique d'éthylène glycol, d'éthylène glycol et d'éther mono-tert-butylique d'éthylène glycol.

4. Procédé selon l'une quelconque des Revendications précédentes selon lequel le rapport molaire de l'éthylène glycol introduit à l'éther di-tert-butylique d'éthylène glycol introduit va de 1:0,01 à 1:20.

5. Procédé selon la Revendication 2 ou 3 selon lequel le rapport molaire de l'éthylène glycol introduit à l'éther di-tert-butylique d'éthylène glycol va de 1:0 à 1:20.

6. Procédé selon les Revendications 2 ou 3 selon lequel le rapport molaire de l'éther di-tert-butylique d'éthylène glycol introduit à l'éther mono-tert-butylique d'éthylène glycol va de 1:0 à 1:5.

7. Procédé selon l'une quelconque des revendications précédentes selon lequel la réaction est effectuée entre 30 et 130°C.

8. Procédé selon la Revendication 1 qui consiste à utiliser, comme éther di-tert-butylique d'éthylène glycol l'éther di-tert-butylique d'éthylène glycol obtenu comme sous-produit de la réaction de l'isobutylène avec l'éthylène glycol en présence d'un échangeur de cations fortement acide comme catalyseur.

9. Procédé selon la Revendication 8 selon lequel ledit sous-produit éther di-tert-butylique d'éthylène glycol est présent en mélange avec au moins un produit choisi parmi l'éther mono-tert-butylique d'éthylène glycol, le diisobutylène et le triisobutylène.

10. Procédé selon la Revendication 9 selon lequel ledit sous-produit éther di-tert-butylique d'éthylène glycol et éliminé dudit mélange sous forme d'azéotrope avec l'éther mono-tert-butylique d'éthylène glycol avant la réaction d'obtention de l'éther mono-tert-butylique d'éthylène glycol.

11. Procédé de préparation de l'éther mono-tert-butylique d'éthylène glycol selon lequel on fait réagir l'éthylène glycol en présence d'un échangeur de cations fortement acide et caractérisé en ce que l'on ajoute préalablement au système réactionnel un additif contenant l'éther di-tert-butylique d'éthylène glycol et que l'on effectue la réaction à une température entre 60 et 130°C.

12. Procédé selon la Revendication 11 selon lequel ledit additif est un mélange d'éther di-tert-butylique d'éthylène glycol et d'éther mono-tert-butylique d'éthylène glycol.

13. Procédé selon la Revendication 12 selon lequel le rapport molaire de l'éther di-tert-butylique d'éthylène glycol à l'éther mono-tert-butylique d'éthylène glycol dans ledit mélange se situe entre 1:0 et 1:5.

14. Procédé selon la Revendication 12, selon lequel le rapport molaire de l'éther di-tert-butylique d'éthylène glycol à l'éther mono-tert-butylique d'éthylène glycol dans ledit mélange se situe entre 1:0 et 1:3.

15. Procédé selon l'une quelconque des Revendications 11 à 14 selon lequel ladite réaction est effectuée en présence de diisobutylène ou de triisobutylène.

16. Procédé selon l'une quelconque des Revendications 11 à 15 selon lequel on utilise, comme composant isobutylène que l'on fait réagir avec l'éthylène glycol, un mélange d'hydrocarbures ayant quatre atomes de carbone et contenant de l'isobutylène.

17. Procédé selon l'une quelconque des Revendications 11 à 16 selon lequel le rapport molaire de l'éthylène glycol à l'isobutylène se situe de 1:0,1 à 1:10.

18. Procédé selon l'une quelconque des Revendications 11 à 17 selon lequel l'éther di-tert-butylique d'éthylène glycol est ajouté dans une quantité d'au moins 10 mole % par rapport à l'éthylène glycol ou à l'isobutylène selon celui qui se trouve en plus faible quantité.

19. Procédé selon l'une quelconque des Revendications 11 à 18 selon lequel l'éther di-tert-butylique de l'éthylène glycol est ajouté dans une quantité de 30 à 80 mole % par rapport à l'éthylène glycol ou à l'isobutylène selon celui qui se trouve en plus faible quantité.

20. Procédé selon l'une quelconque des Revendications 11 à 19 selon lequel ladite température se situe entre 75 et 130°C.

21. Procédé selon l'une quelconque des Revendications 11 à 20 selon lequel ladite température se situe entre 85 et 110°C.

21

22. Procédé selon l'une quelconque des revendications précédentes selon lequel la réaction est effectuée selon un processus discontinu.

23. Procédé selon l'une quelconque des revendications 1 à 21 selon lequel la réaction est effectuée selon un processus continu.

24. Procédé selon la Revendication 23 selon lequel la vitesse linéaire des matériaux de départ dans la réaction est d'au moins 30 cm/hr.

25. Procédé selon l'une quelconque des revendications précédentes selon lequel l'échangeur de cations est un produit sulfoné.

26. Procédé selon la revendication 25, selon lequel l'échangeur de cations est une résine échangeuse de cations type acide phénolsulfonique, un charbon sulfoné ou un asphalte sulfoné.